# EUROPEAN PATENT SPECIFICATION

(11) **EP 4 366 618 B1**
(45) Date of publication and mention of the grant of the patent: **21.01.2026**
(21) Application number: 21948810.3
(22) Date of filing: 08.07.2021
(51) Int. Cl.: A61B 5/145, A61B 5/1473, A61B 5/1486

(54) **MICRO ANALYTE SENSOR**
MIKROANALYTSENSOR
CAPTEUR DE MICRO-ANALYTE

(43) Date of publication of application: 15.05.2024
(73) Proprietor: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2021/105112
(87) International publication number: WO 2023/279312

(56) References cited:
- WO-A1-2021/096403
- CN-A- 104 761 697
- CN-A- 104 825 171
- CN-A- 110 678 122
- CN-A- 111 096 754
- CN-A- 111 742 215
- US-A1- 2016 157 765
- US-A1- 2019 008 425
- US-A1- 2021 145 352

## Description

### TECHNICAL FIELD

The invention mainly relates to the field of medical devices, in particular to a micro analyte sensor.

### BACKGROUND

The pancreas in a normal human body can automatically monitor the level of glucose in the human blood and automatically secrete the required insulin/glucagon. In diabetics, the pancreas does not function properly and cannot produce the insulin the body needs. Therefore, diabetes is a metabolic disease caused by abnormal pancreatic function, and diabetes is a lifelong disease. At present, there is no cure for diabetes with medical technology. The occurrence and development of diabetes and its complications can only be controlled by stabilizing blood glucose.

Diabetics need to have their blood glucose measured before they inject insulin into the body. At present, most of the testing methods can continuously measure blood glucose and send the data to a remote device in real time for the user to view. This method is called Continuous Glucose Monitoring (CGM). The method requires the device to be attached to the skin and the probe it carries is inserted into the tissue fluid beneath the skin.

However, at present, the electronic conduction layer of the sensor electrode is mostly smooth structure, and the adhesion between the electronic conduction layer and the substrate and the membrane layer is small. The electrode is easy to fall off and the membrane layer is easy to shift in the process of use, which affects the reliability and service life of the sensor. In some improved technical schemes, in order to increase the adhesion between the electron conduction layer and the membrane layer, a layer of platinum black is processed on the electron conduction layer, but the adhesion between the platinum black and the substrate is still small, and the processing process of platinum black is complicated and the cost is high.

Therefore, the existing technology urgently needs a micro analyte sensor with strong adhesive force of electrode.

US 2016/157765 A1 describes an implantable biosensor for continuously monitoring an analyte such as glucose. The sensor comprises a substrate with a working electrode, a reference electrode and a counter electrode in one area of the substrate surface. Each electrode is connected to a pad through a wire, respectively. The electrical conducting layer of the working electrode is covered from inside to outside with an anti-interference layer, an enzyme layer, a regulation layer and a biocompatibility layer.

### BRIEF SUMMARY OF THE INVENTION

In view of the disadvantages of existing technology, the present invention implementation example first published in a micro analyte sensor, including at least one electrode group, electrode group includes at least one working electrode and at least one additional electrodes, the working electrode's and/or additional electrode's electrical conduction layer sets at least one surface micro structure, the adhesive force between the electron conduction layer and the substrate and membrane layer can be improved, and the processing cost is lower.

The invention discloses a micro analyte sensor, which comprises a substrate, the substrate comprising an internal part and an external part; at least one electrode group located on a surface of the internal part , wherein the electrode group includes at least one working electrode and at least one additional electrode; pads, wherein the pads corresponding to the working electrode and the additional electrode are arranged in the external part, and the pads are electrically connected with the working electrode and the additional electrode through wires; and at least one surface of an electron conduction layer of the working electrode and/or the additional electrode is provided with a micro structure, wherein the surface of the electron conduction layer attached to the substrate and the surface of the electron conduction layer on the opposite side are provided with a micro structure.

According to one aspect of the invention, the additional electrode includes a counter electrode.

According to one aspect of the invention, the additional electrode also includes a reference electrode.

According to one aspect of the invention, the working electrode, the reference electrode and the counter electrode at least include an electron conduction layer, an anti-interference layer, an enzyme layer, an adjustment layer and a biological compatible layer.

According to one aspect of the invention, the electron conduction layer of the working electrode and the counter electrode is one of graphite, glassy carbon or precious metals.

According to one aspect of the invention, the electron conduction layer of the working electrode and the counter electrode is platinum.

According to one aspect of the invention, the electron conduction layer of the reference electrode is either Ag/AgCl or calomel.

According to one aspect of the invention, the micro structure includes either a micro-groove or a micro-bulge.

According to one aspect of the invention, the micro-groove includes one or more of a micro-through-hole or a micro-blind hole or a micro-notch.

According to one aspect of the invention, the micro-structure has a diameter of 0. 001-100um.

According to one aspect of the invention, the density of the micro structure is 1*10²∼1*10¹⁰/cm².

According to one aspect of the invention, the material of the substrate is selected from one or more combinations of polytetrafluoroethylene, polyethylene, polyvinyl chloride, acrylonitrile-butadiene-styrene copolymer, polymethyl methacrylate, polycarbonate and polyimide.

Compared with the prior art, the technical scheme of the invention has the following advantages:
The micro analyte sensor is provided with a working electrode and an additional electrode in the internal part of the substrate, and all the electrodes are electrically connected with the corresponding pad arranged in the external part through a wire. At least one surface of the electronic conduction layer of the working electrode and/or the additional electrode is provided with a micro structure to increase the surface area and roughness of the electronic conduction layer, enhance the adhesion force of the electrode and improve the detection reliability of the sensor , so it can increase adhesive force between the electrical conduction layer and substrate, membrance layer, thus it reduces the possibility that the electrode and the membrance layer may shift or fall off during use, improve the reliability of the sensor.

Further, the micro analyte sensor disclosed in the invention can be divided into a three-electrode system and a two-electrode system, wherein the three-electrode system consists of a counter electrode, a reference electrode and at least one working electrode, and the two-electrode system consists of a counter electrode and at least one working electrode. In addition, according to the number of working electrodes, the invention can also be divided into two situations: 1) single working electrode: there is only one working electrode;2) Dual working electrode: there are two working electrodes, one of which is called "working electrode" for electroredox reaction with analyte to generate electrical signal, and the other is usually responsible for detecting the response signal of interference or background solution, called "auxiliary electrode". All the electrode composition methods mentioned above have their own unique advantages, among which the three-electrode system can effectively control the detection potential, prevent potential drift, and improve the reliability of the parameter information of the detection analyte. However, the two-electrode system has simple structure and lower production cost.

Furthermore, the electron conduction layer of each electrode is made of materials with good conductivity and strengthening inert as the electrode material. One of graphite, glassy carbon or precious metals, one of Ag/AgCl or calomel as a reference electrode of the preferred working electrode and counter electrode. Considering the requirement of good ductility and stability of surface structure, precious metal materials such as gold, platinum and silver become the better choice. Preferably, both the working electrode and the counter electrode are platinum.

Further, the electrical conduction layer on the surface of the micro structure can be micro grooves, can also be a tiny bumps, when the micro structure is a micro grooves, can be a tiny hole or tiny blind holes or micro indentation, one or more of that micro structures are not limited to its own specific shape, can increase the surface area and roughness, reduce the requirements of processing technology and processing costs.

Further, the substrate material of the sensor is selected from one or more combinations of polytetrafluoroethylene (TEFLON), polyethylene (PE), polyvinyl chloride (PVC), acrylonitrile-butadiene-styrene copolymer (ABS), polymethyl methacrylate (PMMA), polycarbonate (PC), polyimide (PI), etc. All of the above materials have excellent insulating properties, water impermeability and high mechanical strength, which can extend the service life of the sensor.

The second aspect of the present invention discloses a continuous analyte monitoring device, which comprises a bottom shell for mounting on the skin surface of the host. The sensor unit comprises a base and at least one micro analyte sensor as described above. The micro analyte sensor is fixed on the base, and the sensor unit is installed on the bottom shell through the base to detect the analyte parameter information in the host body. The transmitter is electrically connected with the sensor unit for sending the parameter information of the analyte to the outside world. A battery, which is used to provide electrical energy. And a receiver, which is used to receive analyte parameter information and indicate to the user.

The reliability of the sensor is often the key factor limiting the reliability of the continuous analyte monitoring device. By setting a micro structure on the surface of the electrode electron conduction layer, the adhesion force between the electron conduction layer, the substrate and the membrance layer is enhanced to improve the reliability of the sensor, and therefore the reliability of the continuous analyte monitoring device is also improved.

### BRIEF DESCRIPTION OF THE DRAWINGS

Fig. 1 is a structural schematic diagram of a sensor according to an embodiment of the invention;
Fig. 2 is a side view of the sensor in the embodiment of Fig. 1;
Fig. 3 shows a sectional view of the electrode;
Fig. 4 is a schematic diagram of the micro structure of the surface of the electron conduction layer according to an embodiment of the invention;
Fig. 5 is the V-V 'profile of Fig. 4;
Fig. 6 is a schematic diagram of a continuous analyte monitoring device according to an embodiment of the invention.

### DETAILED DESCRIPTION

As mentioned above, the adhesion between the electrode electron conduction layer and the substrate and the membrance layer of the analyte sensor with the existing technology is weak, and the electrode and the membrance layer are easy to shift or fall off during the use, which affects the reliability of the sensor.

In order to solve this problem, the invention provides a micro analyte sensor, which is arranged on the surface of the internal part on the substrate with at least one electrode group, the electrode group comprises at least one working electrode and at least one additional electrode, and all the electrodes are electrically connected with the corresponding PAD arranged on the internal part through a wire. The working electrode's and the additional electrode's electrical conduction layer surface is set with micro structure, so as to increase the surface area of electrical conduction layer at the same time, also increased the roughness of the surface, therefore, the adhesive force between the electron conduction layer, the substrate and the membrance layer can be increased, which reduces the possibility of the electrode and the membrance layer shifting or falling off in the process of use, and improves the reliability of the sensor .

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the present invention.

In addition, it should be understood that the dimensions of the various components shown in the attached drawings are not necessarily drawn to actual proportions for ease of description, e. g. the thickness, width, length or distance of some elements may be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once an item is defined or described in one of the appending drawings, there is no need to discuss it further in the subsequent appending drawings.

In addition, it should be understood that one or more method steps referred to in the present invention do not exclude the possibility that other method steps may exist before and after the combined steps or that other method steps may be inserted between such explicitly mentioned steps, unless otherwise stated. It should also be understood that the combination connection between one or more devices/devices referred to in the invention does not preclude the existence of other devices/devices before and after the said combination devices or the insertion of other devices between the two specifically mentioned devices, unless otherwise stated. And, unless otherwise specified, the serial number of the steps just a convenient tool for identifying the steps, rather than to limit the steps of the order or limit the scope of the present invention can be implemented, the relationship of the relative change or adjust, in the case of no substantial changes to technical content, when as well as the category of the present invention can be implemented.

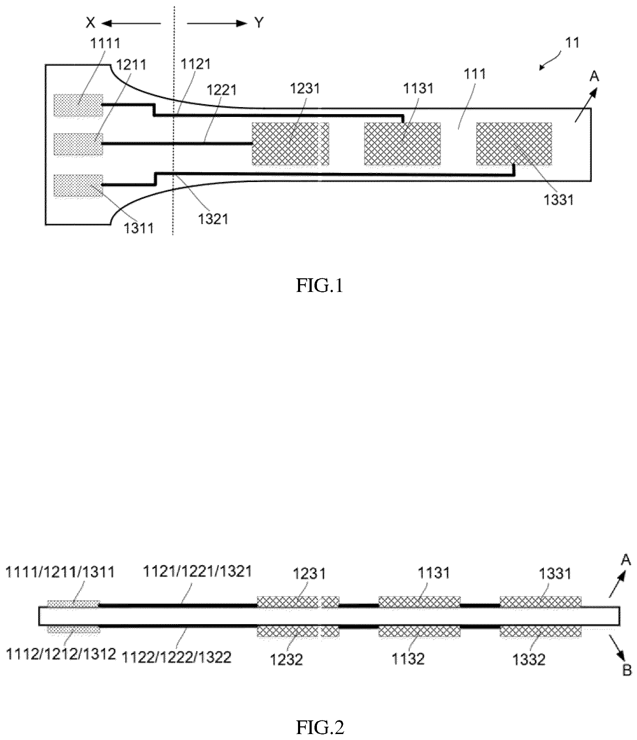

Fig. 1 is the structural schematic diagram of the sensor in the embodiment of the invention;Fig. 2 is a side view of the sensor in the embodiment of Fig. 1.

Sensor 11 includes the base 111, which is divided into an external part X and an internal part Y as shown in Fig. 1 with dotted lines as the dividing line. Spread with the internal part Y electrode, including at least one of the working electrode 1131 and at least one additional electrodes, obviously, in this example, additional electrodes include counter electrode 1231 and reference electrode 1331, which constitute three-electrodes system, the counter electrode 1231 is opposite to the working electrode 1131, it forms a closed loop with the working electrode 1131, so that the current on the electrode can conduct normally. The reference electrode 1331 is used to provide the reference potential of the working electrode 1131, so that the detection potential can be effectively controlled. In this invention in another example, additional electrodes may also include counter electrode 1231 only, so as to form a two-electrode system, compared to the three-electrode system, the effective area of working electrode 1131 and counter electrode 1231 can be increased on the limited area of internal part Y, so as to prolong the service life of the electrode, and because one electrode is removed, the process is simpler. However, working electrode 1131 does not have the detection potential of the reference electrode as a reference, so the reliability of the detection information of the analyte will be reduced. In another embodiment of the present invention, the working electrode 1131 has at least two, one of which generates an electrical signal by electroredox reaction with the analyte to be detected, and the other is used to detect the response signal of interference or background solution in the body fluid of the host, which called auxiliary electrode.

Continuously refer to Fig. 1 and Fig. 2, the external part X is provided with a PAD , which corresponds to the electrode one-to-one and is electrically connected through a wire, that is, the first PAD1111 corresponding to the working electrode 1131 is electrically connected through wire 1121, the second PAD1211 corresponding to the counter electrode 1231 is electrically connected through wire 1221, and the third PAD1311 corresponding to the reference electrode 1331 is electrically connected through wire 1321. The different pads, wires, and electrodes are insulated from each other to prevent interference with electrical signals.

Since sensor 11 is of planar structure, there are two opposite surfaces, namely surface A and surface B. The working electrode 1131, the counter electrode 1231 and the reference electrode 1331 are laid on the A surface of the sensor as an electrode group. In contrast, on the sensor's B, laid another electrode group, the electrode configuration can be a two-electrode system, can also be a three-electrode system, also can be double working electrode, optimization, consistent with surface A , which includes working electrode 1132, counter electrode 1232 and reference electrode 1332, the same, also laid on the surface of the B has PAD,the PAD corresponds to the electrode on surface B one-to-one, and is electrically connected through a wire, that is, the fourth PAD 1112 corresponding to the working electrode 1132 is electrically connected through wire 1122. The fifth PAD 1212 corresponding to the counter electrode 1232 is electrically connected through wire 1222. And the sixth Pad 1312 corresponding to the reference electrode 1332, electrically connected through wire 1322. In this way, if any electrode on surface A terminates its life or fails in advance, the same electrode on surface B can take over and enter the working state, improving the reliability of the parameter data of the detection analyte and prolonging the service life of the sensor.

Technicians in this field should understand that there is no restriction on the sequence or position of the pads, conductors and electrodes laid on either surface A or B of the sensor. The pads, wires, and electrodes on both surfaces may be symmetrically or asymmetrically arranged. The corresponding PAD, wire and electrode are laid on the same surface or can be laid on different surfaces. Preferably, the corresponding PAD, wire and electrode are laid on the same surface to facilitate the wiring of the wire. For example, the position of the working electrode 1131 on surface A can be changed with that of the counter electrode 1231 on surface A, or the position of the counter electrode 1231 on surface A can be changed with that of the reference electrode 1332 on plane B. No matter how the order and position of the PAD, wire and electrode on surface A and B change,As long as the PAD, wire and electrode exist one - to - one correspondence, each other can be insulated.

In other embodiments of the present invention, the service life of the sensor can be further extended by increasing the number of electrode groups by increasing the sensor area or decreasing the electrode area, although the planar structure sensor only has relative surface A and surface B. However, too large sensor area may increase the host's rejection reaction and cause the host's discomfort. Too small electrode area will reduce the sensitivity of the electrode and reduce the reliability of the detection parameters. An excessive number of electrode groups will also increase the complexity of the processing process, for example, the wiring of the wire will become very dense. Therefore, it is preferred that the number of electrode groups be two.

In other embodiments of the present invention, each electrode group may also be distributed on the same surface of the sensor, such as surface A or surface B, without limitation herein.

In the embodiment of the invention, the substrate 111 is a material with excellent insulating properties, mainly from inorganic non-metallic ceramics, silica glass and organic polymers, etc. At the same time, considering the application environment of implantable electrode, the substrate material is also required to have high water permeability and mechanical strength. Preferately, the substrate materials are selected from one or more combinations of polytetrafluoroethylene (Teflon), polyethylene (PE), polyvinyl chloride (PVC), acrylonitrile-butadiene-styrene copolymer (ABS), polymethyl methacrylate (PMMA), polycarbonate (PC), polyimide (PI), etc.

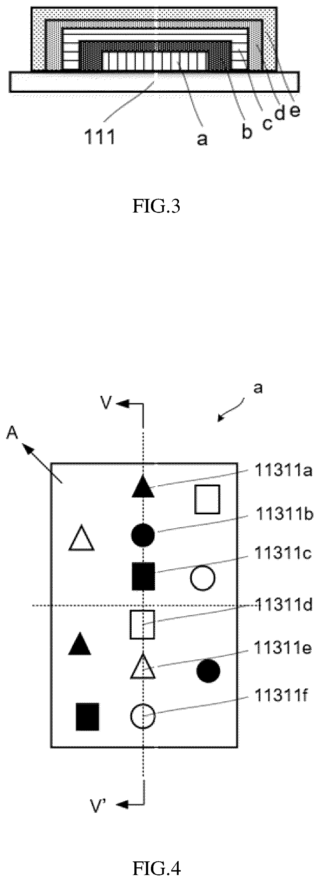

Fig. 3 shows a sectional view of the electrode. In one embodiment of the invention, the working electrode (auxiliary electrode), the counter electrode and the reference electrode at least include the electron conduction layer a, the anti-interference layer b, the enzyme layer c, the adjustment layer d and the biological compatibility layer e. And the anti-interference layer b, the enzyme layer c, the adjustment layer d and the biological compatibility layer e are collectively referred to as the membrane layer.

### Electron conduction layer:

The electron conduction layer a is made of materials with good electrical conductivity and fortification inertia. Preferred, the working electrode and the counter electrode are selected from graphite electrode, glass carbon electrode, noble metal and other materials, the reference electrode is selected from one of Ag/AgCl or calomel. Considering the requirements of good ductility and stability of surface structure, noble metal electrodes, such as gold electrode, platinum electrode and silver electrode, become a better choice. The working electrode and counter electrode are both platinum electrode for further optimization.

In the patented technical solution, the micro structure is arranged in the electron conduction layer a.

### Anti-interference layer:

The anti-interference layer b is located between the enzyme layer and the electron conduction layer. Interferers are molecules or substances that undergo electrochemical reduction or oxidation on the electrode surface, either directly or indirectly through an electron transfer agent, resulting in an erroneous signal that interferes with analyte detection. For example, for the determination of glucose as an analyte, common interferences in the body are urea, ascorbic acid, acetaminophen, and so on.

In the preferred example, the anti-interference layer b prevents one or more interference agents from penetrating the electrolyte surrounding the electrode. For example, the anti-interference layer b allows the analyte to be measured at the electrode (e. g. , hydrogen peroxide) to pass through, while at the same time preventing the passage of other substances (e. g. , potentially interfering substances). In a preferred scenario, the anti-interference layer b could be a very thin membrance designed to limit the diffusion of substances with molecular weights greater than 34Da.

In another preferred example, the anti-interference layer b can be an organic polymer, which can be prepared from organosilane and a hydrophilic copolymer. Hydrophilic copolymers, preferably, polyethylene glycol (PEG), poly (2-hydroxyethyl methacrylate) and poly (lysine). In a preferred embodiment, the thickness of the anti-interference layer b may range from 0. 1 um or less to 10 um or more. The preferred thickness range is 0. 5 um to 5 um.

### Enzyme layer:

The enzyme layer c is coated with active enzymes. According to the type of analyte to be detected, the corresponding active enzymes are coated. Active enzymes can make the analyte to be detected produce some chemical reactions and generate electrons. According to different concentrations of analyte to be detected, the number of electrons produced is different, and the electrons are collected by the electron conduction layer, thus forming different current intensity. Therefore, current intensity information can be used to characterize the parameter information of the analyte.

Preferably, the enzyme layer c is coated with glucose oxidase (GOx).

### Adjustment layer:

The adjustment layer d is located above the enzyme layer. In the embodiment of the present invention, when the enzyme layer is coated with glucose oxidase, the adjustment layer d is mainly used to regulate the transmittance of oxygen and glucose transferred to the enzyme layer. The amount of glucose (molar concentration) in body fluids is one order of magnitude higher than the amount of oxygen. However, for enzymatic sensors that require oxygen, an excess oxygen supply is needed to ensure that oxygen does not become a limiting substance, so that the sensor can respond linearly to changes in glucose concentration without being affected by oxygen partial pressure. In other words, when oxygen content is the limiting factor, the linear range of glucose oxygen monitoring reaction does not reach the expected concentration range. Without a semi-permeable membrane above the enzyme layer to regulate the passage of oxygen and glucose, the upper limit of the sensor's linear response to glucose is only about 40mg/dL. However, in a clinical setting, the upper limit of the linear response of blood glucose levels needs to be about 500mg/dL.

Adjustment layer d acts primarily as a semi-permeable membrane to regulate the amount of oxygen and glucose transmitted to the enzyme layer and, more specifically, to make oxygen excess a non-limiting factor. The upper limit of the linear response of the sensor to glucose with the adjustment layer can be reached to a higher level than that without the adjustment layer. In a preferred example, the ratio of oxygen-glucose transmittance in adjustment layer d can be reached to 200:1, thus ensuring that sufficient oxygen is available for the enzymatic reaction at any glucose and oxygen concentration that may be present subcutaneally.

In one preferred example, the adjustment layer d may be an organic polymer, which may be prepared from organosilane and a hydrophilic copolymer. Hydrophilic copolymer, preferably, copolymerization or graft of polyethylene glycol (PEG). Other hydrophilic copolymers that may be used include, but are not limited to, other diols such as propylene glycol, esters, amides, carbonates, and polypropylene glycol. The use of organosilicone polymers can obviously improve the oxygen transmission, and effectively control the glucose transmission. In a preferred implementation, the adjustment layer d may be in the thickness range of 1 um or less to 50 um or greater, with a preferred thickness range of 1 um to 10 um.

### Biological compatibility layer:

The biocompatibility layer e is located at the outermost part of the electrode, which is designed to eliminate the body's rejection of foreign bodies and reduce the formation of a shielding cell layer around the implanted electrode.

In a preferred example, the biocompatibility layer e can be prepared from organosilanes and a hydrophilic copolymer. Hydrophilic copolymer, preferably, copolymerization or graft of polyethylene glycol (PEG). Other hydrophilic copolymers that may be used include, but are not limited to, other diols such as propylene glycol, esters, amides, carbonates, and polypropylene glycol.

In a preferred embodiment, the thickness of the biocompatibility layer e may range from 1 micron or less to 100 microns or more. A preferred thickness range is 10 um to 30 um.

In the embodiment of the invention, the thickness of base 11 is 0. 01~0. 8mm, each electrode is rectangular, the width of each electrode is 0. 01~1mm, and the area is 0. 1~2mm².

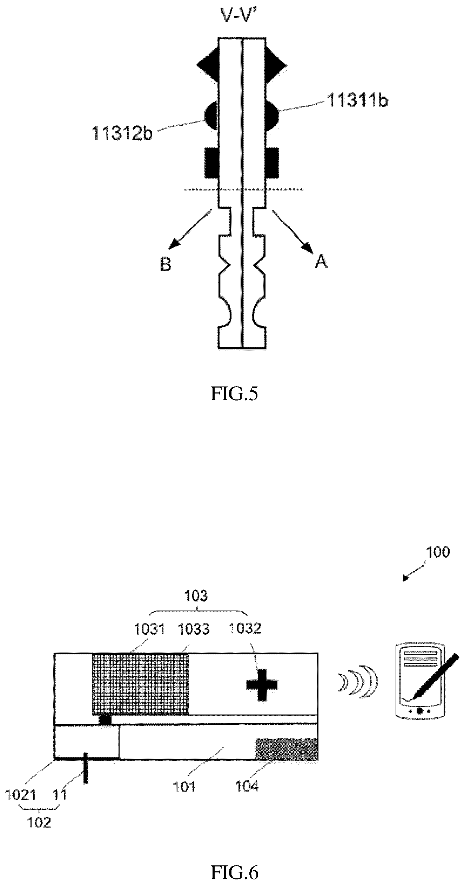

Fig. 4 is a schematic diagram of the electrode surface micro-structure of an embodiment of the invention, and Fig. 5 is the V-V 'profile of Fig. 4.

In the embodiment of the invention, taking the working electrode 1131 as an example, the electronic conduction layer a of the electrode is divided into surface A and surface B. One side is attached to the base 11 of the sensor, and the other side is attached to the membrane layer (b \ c \ d \ e). There are micro structures on both sides, which can increase the roughness of the surface on the other hand, in order to enhance the electrical conduction layer a and a base 11, membrane layer (b \ c \ d \ e), the adhesion force, prevent when use, the electrode on the basal or membrane layer between shift or fall off, at the same time also can enhance the electrode with polymer, such as the adhesion strength of the glucose in the body, improve the reliability and stability of the sensors.

The micro structures protruding outside the surface of the electron conduction layer A form micro bulges, such as micro bulges 11311a, 11311b, 11311c, etc. The micro bulges have no specific shape restrictions, and can be tri-prismatic, spherical, rectangular or irregular, with a diameter of 0. 001~100um, which is preferred. The diameter is 10~50um.

Accordingly, micro-grooves are formed by concave micro-structures inside the surface of electron conduction layer a, such as micro-grooves 11311d, 11311e and 11311f. There is no specific shape restriction for the micro-bulges, which can be tri-prismatic, spherical, rectangular or irregular. Shapes such as the notch of a strip (not shown). The diameter of the micro grooves is 0. 001~100um, and the height of the micro bulge or the depth of the micro grooves is 0. 001~50um. Preferably, the diameter is 10~50um, the height of the micro bulge or the depth of the micro groove is 10~30um.

In the embodiments of the invention, no matter the micro bulges or micro grooves, on either side of the electron conduction layer a, they can be evenly arranged according to the interval of equal height or equal width, or they can be randomly arranged, and no restriction is made herein. However, its density needs to be limited to a certain range, such as 1*10²~1*10¹⁰/cm², too large or too small of the micro structure density can not achieve the desired effect of increasing adhesion force.

In embodiments of the present invention, micro bulges or micro grooves may be obtained by means of chemical etching. For example, through the process of cleaning - chemical solution (HCl/C₂H₂O₂/NH₄Cl/FeCl₃) corrosion - cleaning - brightening treatment - cleaning - drying treatment, can be obtained about 1~50 um in diameter, density 5*10²~3*10³/cm², thickness of 1~10 um micro convex or micro groove. For another example, the laser etching process can also be used to control the laser stroke and energy to obtain the micro bump or micro groove as the above parameters. The process of producing micro-grooves or micro-bulges is simpler and less costly than that of producing platinum black.

By chemical etching method to get the micro structure of irregular shape, according to the amount of chemical liquid and corresponding processing technology can be respectively hole or blind hole or nicks, aperture within a certain range of change, each hole diameter also vary in different directions, the irregularly shaped hole is more conducive to improving the adhesion between the electron conduction layer and the substrate and membrane layer.

It is understood by those skilled in this field that the processes used to produce the micro-grooves or micro-bumps mentioned above are not limited to the processes mentioned above, and that any process that can be used to produce the micro-grooves or micro-bumps by micromachining can be used.

In other embodiments of the present invention, the electron conduction layer a is also provided with a carbon nanotube modified layer (not shown in the figure). Using the unique mechanical strength, high specific surface area, fast electron transfer effect and chemical stability of carbon nanotubes, the carbon nanotubes are modified to the surface of the electron conduction layer a on the surface of the formed electron conduction layer a by means of physical adsorption, embedding or covalent bonding to improve the electron transfer speed. At the same time, because of its large specific surface area, it can be used as an excellent catalyst (enzyme) carrier. The modified carbon nanotube layer can be fixed on the surface of the electron conduction layer a by Nafion solution dispersion method, covalent fixation method, etc.

Fig. 6 is a schematic diagram of the continuous analyte monitoring device 100 in an embodiment of the invention. The continuous analyte monitoring device 100 includes a chassis 101 for mounting on the skin surface of the host;The sensor unit 102 comprises a base 1021 and a micro analyte sensor 11 as described above. The micro analyte sensor 11 is fixed on the base, and the flyer unit 102 is installed on the bottom shell 101 through the base;The transmitter unit 103 comprises an internal circuit 1031, an transmitter 1032 and an electrical connection area 1033. The electrical connection area 1033 is electrically connected with the sensor unit 102. The internal circuit 1031 stores the predetermined conditions of the electrode switching described above, and the transmitter 1032 is used to send the analytical parameter information to the outside world. Battery 104, battery 104 is used to provide electricity;Receiver 105, Receiver 105 is used to receive analyte parameter information and indicate to the user.

In summary, the invention discloses a micro analyte sensor, which is provided with at least one electrode group arranged on the surface of the internal part on the substrate, and the electrode group comprises at least one working electrode and at least one additional electrode. All the electrodes are electrically connected with the corresponding PAD arranged in the internal part through a wire. Working electrode and the additional electrical conduction layer surface of the electrode is set with micro structure, increase the surface area of electrical conduction layer at the same time, also increased the roughness of the surface, therefore, the adhesion force between the electron conduction layer and the substrate and membrane layer can be increased, it reduces the possibility of electrode and membrance layer shifting or falling off in the process of use and improves the reliability of the sensor .

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope of the present invention. The scope of the invention is limited by the attached claims.

## Claims

1. A micro analyte sensor (11), comprising
a substrate (111) comprising an internal part (Y) and an external part (X);
at least one electrode group, located on a surface of the internal part (Y), wherein the electrode group comprises at least one working electrode (1131) and at least one additional electrode (1231, 1331);
pads (1111, 1211, 1311), wherein the pads corresponding to the working electrode (1131) and the additional electrode (1231, 1331), are arranged in the external part (X), and the pads (1111, 1211, 1311) are electrically connected with the working electrode (1131) and the additional electrode (1231, 1331) through wires (1121, 1221, 1321); and at least one surface of an electron conduction layer (a) of the working electrode (1131) and/or the additional electrode (1231, 1331) is provided with a micro structure (11311),
**characterized in, that**
the surface (B) of the electron conduction layer (a) attached to the substrate (111) and the surface (A) of the electron conduction layer (a) on the opposite side are provided with a micro structure (11312b, 11311b).

2. The micro analyte sensor according to claim 1, wherein the additional electrode includes a counter electrode (1231).

3. The micro analyte sensor according to claim 2, wherein the additional electrode includes a reference electrode (1331).

4. The micro analyte sensor according to claim 3, wherein each of the working electrode (1131), the reference electrode (1331) and the counter electrode (1231) at least include an electron conduction layer (a), an anti-interference layer (b), an enzyme layer (c), an adjustment layer (d) and a biological compatible layer (e).

5. The micro analyte sensor according to claim 4, wherein each of the electron conduction layer (a) of the working electrode (1131) and the electron conduction layer (a) of the counter electrode (1231) is one of graphite, glassy carbon or noble metal.

6. The micro analyte sensor according to claim 5, wherein each of the electron conduction layer (a) of the working electrode (1131) and the electron conduction layer (a) of the counter electrode (1231) is platinum.

7. The micro analyte sensor according to claim 4, wherein the electron conduction layer (a) of the reference electrode (1331) is one of Ag/AgCl or calomel.

8. The micro analyte sensor according to claim 1, it is characterized that the micro structure (11311) includes micro grooves (11311d, 11311e, 11311f) or micro bulges (11311a, 11311b, 11311c).

9. The micro analyte sensor according to claim 8, wherein the micro grooves (11311d, 11311e, 11311f) include at least one of micro through holes, micro blind holes and micro notches.

10. The micro analyte sensor according to claim 1, wherein a diameter of the micro structure (11311) is 0. 001~100um.

11. The micro analyte sensor according to claim 1, wherein a density of the micro structure (11311) is 1*10²∼1*10¹⁰/cm².

12. The micro analyte sensor according to claim 1, wherein the substrate (111) comprises one or more combinations of polytetrafluoroethylene, polyethylene, polyvinyl chloride, acrylonitrile-butadiene-styrene copolymer, polymethyl methacrylate, polycarbonate and polyimide.

13. A continuous analyte monitoring device (100), comprising:
a bottom shell (101) installed on ta surface of a host;
a sensor unit (102) comprising a base (1021) and at least one micro analyte sensor (11) according to claim 1, wherein the micro analyte sensor (11) is fixed on the base (1021) and the sensor unit (102) is installed on the bottom shell (101) through the base (1021) to detect analyte parameter information in the host;
a transmitter (1032) electrically connected with the sensor unit (102) for outputting the analyte parameter information;
a battery (104) configured to provide electric energy; and
a receiver configured to receive the analyte parameter information and to display.

## Patentansprüche

1. Ein Mikroanalytsensor (11), umfassend
ein Substrat (111) mit einem inneren Teil (Y) und einem äußeren Teil (X);
mindestens eine Elektrodengruppe, die sich auf einer Oberfläche des inneren Teils (Y) befindet, wobei die Elektrodengruppe mindestens eine Arbeitselektrode (1131) und mindestens eine zusätzliche Elektrode (1231, 1331) umfasst;
Pads (1111, 1211, 1311), wobei die Pads, die der Arbeitselektrode (1131) und der zusätzlichen Elektrode (1231, 1331) entsprechen, im äußeren Teil (X) angeordnet sind und die Pads (1111, 1211, 1311) über Drähte (1121, 1221, 1321) elektrisch mit der Arbeitselektrode (1131) und der zusätzlichen Elektrode (1231, 1331) verbunden sind; und mindestens eine Oberfläche einer Elektronenleitungsschicht (a) der Arbeitselektrode (1131) und/oder der zusätzlichen Elektrode (1231, 1331) mit einer Mikrostruktur (11311) versehen ist,
**dadurch gekennzeichnet, dass**
die Oberfläche (B) der an dem Substrat (111) befestigten Elektronenleitungsschicht (a) und die Oberfläche (A) der Elektronenleitungsschicht (a) auf der gegenüberliegenden Seite mit einer Mikrostruktur (11312b, 11311b) versehen sind.

2. Der Mikroanalytsensor nach Anspruch 1, wobei die zusätzliche Elektrode eine Gegenelektrode (1231) umfasst.

3. Der Mikroanalytsensor nach Anspruch 2, wobei die zusätzliche Elektrode eine Referenzelektrode (1331) umfasst.

4. Der Mikroanalytsensor nach Anspruch 3, wobei die Arbeitselektrode (1131), die Referenzelektrode (1331) und die Gegenelektrode (1231) jeweils mindestens eine Elektronenleitungsschicht (a), eine Anti-Interferenzschicht (b), eine Enzymschicht (c), eine Anpassungsschicht (d) und eine biologisch kompatible Schicht (e) umfassen.

5. Der Mikroanalytsensor nach Anspruch 4, wobei sowohl die Elektronenleitungsschicht (a) der Arbeitselektrode (1131) als auch die Elektronenleitungsschicht (a) der Gegenelektrode (1231) aus Graphit, Glaskohlenstoff oder Edelmetall besteht.

6. Der Mikroanalytsensor nach Anspruch 5, wobei sowohl die Elektronenleitungsschicht (a) der Arbeitselektrode (1131) als auch die Elektronenleitungsschicht (a) der Gegenelektrode (1231) aus Platin besteht.

7. Der Mikroanalytsensor nach Anspruch 4, wobei die Elektronenleitungsschicht (a) der Referenzelektrode (1331) aus Ag/AgCl oder Kalomel besteht.

8. Der Mikroanalytsensor nach Anspruch 1, **dadurch gekennzeichnet, dass** die Mikrostruktur (11311) Mikrorillen (11311d, 11311e, 11311f) oder Mikroausbuchtungen (11311a, 11311b, 11311c) umfasst.

9. Der Mikroanalytsensor nach Anspruch 8, wobei die Mikrorillen (11311d, 11311e, 11311f) mindestens eines von Mikro-Durchgangslöchern, Mikro-Sacklöchern und Mikro-Kerben umfassen.

10. Der Mikroanalytsensor nach Anspruch 1, wobei der Durchmesser der Mikrostruktur (11311) 0,001 bis 100 um beträgt.

11. Der Mikroanalytsensor nach Anspruch 1, wobei die Dichte der Mikrostruktur (11311) 1*10² ~ 1*10¹⁰/cm² beträgt.

12. Der Mikroanalytsensor nach Anspruch 1, wobei das Substrat (111) eine oder mehrere Kombinationen aus Polytetrafluorethylen, Polyethylen, Polyvinylchlorid, Acrylnitril-Butadien-Styrol-Copolymer, Polymethylmethacrylat, Polycarbonat und Polyimid umfasst.

13. Vorrichtung (100) zur kontinuierlichen Überwachung von Analyten, umfassend:
ein Bodengehäuse (101), die auf einer Oberfläche eines Hosts installiert ist;
eine Sensoreinheit (102) mit einer Basis (1021) und mindestens einen Mikroanalytsensor (11) nach Anspruch 1, wobei der Mikroanalytsensor (11) an der Basis (1021) befestigt ist und die Sensoreinheit (102) über die Basis (1021) an der unteren Hülle (101) angebracht ist, um Analytparameterinformationen im Host zu erfassen;
einen Sender (1032), der elektrisch mit der Sensoreinheit (102) verbunden ist, um die Analytparameter auszugeben;
eine Batterie (104), die zur Bereitstellung von elektrischer Energie konfiguriert ist; und
einen Empfänger, der so konfiguriert ist, dass er die Analytparameterinformationen empfängt und anzeigt.

## Revendications

1. Capteur de micro-analyte (11), comprenant
un substrat (111) comprenant une partie interne (Y) et une partie externe (X) ;
au moins un groupe d'électrodes, situé sur une surface de la partie interne (Y), dans lequel le groupe d'électrodes comprend au moins une électrode de travail (1131) et au moins une électrode supplémentaire (1231, 1331) ;
des plots (1111, 1211, 1311), dans lequel les plots correspondant à l'électrode de travail (1131) et à l'électrode supplémentaire (1231, 1331), sont agencés dans la partie externe (X), et les plots (1111, 1211, 1311) sont raccordés électriquement à l'électrode de travail (1131) et à l'électrode supplémentaire (1231, 1331) via des câbles (1121, 1221, 1321) ; et au moins une surface d'une couche de conduction d'électron (a) de l'électrode de travail (1131) et/ou de l'électrode supplémentaire (1231, 1331) est munie d'une micro structure (11311), **caractérisé en ce que**
la surface (B) de la couche de conduction d'électron (a) attachée au substrat (111) et la surface (A) de la couche de conduction d'électron (a) sur le côté opposé sont munies d'une micro structure (11312b, 11311b).

2. Capteur de micro-analyte selon la revendication 1, dans lequel l'électrode supplémentaire inclut une contre-électrode (1231).

3. Capteur de micro-analyte selon la revendication 2, dans lequel l'électrode supplémentaire inclut une électrode de référence (1331).

4. Capteur de micro-analyte selon la revendication 3, dans lequel chaque électrode de travail (1131), électrode de référence (1331) et contre-électrode (1231) inclut au moins une couche de conduction d'électron (a), une couche anti-interférences (b), une couche enzymatique (c), une couche d'ajustement (d) et une couche biologiquement compatible (e).

5. Capteur de micro-analyte selon la revendication 4, dans lequel chaque couche de conduction d'électron (a) de l'électrode de travail (1131) et couche de conduction d'électron (a) de la contre-électrode (1231) est soit en graphite, soit en carbone vitreux ou en métal noble.

6. Capteur de micro-analyte selon la revendication 5, dans lequel chaque couche de conduction d'électron (a) de l'électrode de travail (1131) et couche de conduction d'électron (a) de la contre-électrode (1231) est en platine.

7. Capteur de micro-analyte selon la revendication 4, dans lequel la couche de conduction d'électron (a) de l'électrode de référence (1331) est soit en Ag/AgCl, soit en calomel.

8. Capteur de micro-analyte selon la revendication 1, **caractérisé en ce que** la micro-structure (11311) inclut des micro-rainures (11311d, 11311e, 11311f) ou des micro-bosses (11311a, 11311b, 11311c).

9. Capteur de micro-analyte selon la revendication 8, dans lequel les micro-rainures (11311d, 11311e, 11311f) incluent au moins un élément parmi les micro-trous traversants, les micro-trous borgnes et les micro-encoches.

10. Capteur de micro-analyte selon la revendication 1, dans lequel le diamètre de la micro-structure (11311) est de 0,001-100 um.

11. Capteur de micro-analyte selon la revendication 1, dans lequel la densité de la micro-structure (11311) est de 1*10²∼1*10¹⁰/cm².

12. Capteur de micro-analyte selon la revendication 1, dans lequel le substrat (111) comprend une ou plusieurs combinaisons de polytétrafluoroéthylène, de polyéthylène, de chlorure de polyvinyl, de copolymère d'acrylonitrile-butadiène-styrène, de méthacrylate de polyméthyle, de polycarbonate et de polyimide.

13. Dispositif de surveillance continue d'analyte (100), comprenant :
une coque inférieure (101) installée sur la surface d'un hôte ;
une unité de capteur (102) comprenant une base (1021) et au moins un capteur de micro-analyte (11) selon la revendication 1, dans lequel le capteur de micro-analyte (11) est fixé sur la base (1021) et l'unité de capteur (102) est installée sur la coque inférieure (101) à travers la base (1021) pour détecter les informations de paramètres d'analyte dans l'hôte ;
un émetteur-récepteur (1032) raccordé électriquement à l'unité de capteur (102) pour produire les informations de paramètres d'analyte ;
une batterie (104) configurée pour fournir une énergie électrique ; et
un récepteur configuré pour recevoir les informations de paramètres d'analyte et les afficher.
